# EUROPEAN PATENT SPECIFICATION

(11) **EP 4 050 094 B1**
(45) Date of publication and mention of the grant of the patent: **21.02.2024**
(21) Application number: 19940165.4
(22) Date of filing: 26.11.2019
(51) Int. Cl.: C12N 1/20, B09C 1/10, C12R 1/01, C12N 11/02

(54) **EFFICIENT PETROLEUM DEGRADATION BACTERIA TDYN1T AND USE THEREOF**
EFFIZIENTE ERDÖLABBAUBAKTERIEN TDYN1T UND DEREN VERWENDUNG
BACTÉRIES TDYN1T EFFICACES POUR LA DÉGRADATION DU PÉTROLE ET LEUR UTILISATION

(30) Priority: 21.10.2019 CN 201910997997
(43) Date of publication of application: 31.08.2022
(73) Proprietor: Tianjin University, Tianjin 300072 (CN)
(72) Inventor: CHEN, Guanyi, Tianjin 300072 (CN); ZHONG, Lei, Tianjin 300072 (CN); TIAN, Jingnan, Tianjin 300072 (CN); TIAN, Shu, Tianjin 300072 (CN); SUN, Yuru, Tianjin 300072 (CN); WU, Wenzhu, Tianjin 300072 (CN); MA, Wenchao, Tianjin 300072 (CN)
(74) Representative: Cabinet Beau de Loménie
(86) International application number: PCT/CN2019/120938
(87) International publication number: WO 2021/077531

(56) References cited:
- CN-A- 104 560 777
- CN-A- 104 805 044
- CN-A- 104 805 044
- CN-A- 108 467 842
- CN-A- 108 949 634
- CN-B- 104 651 280
- CN-B- 106 434 470
- JP-A- 2013 031 402
- JP-A- 2013 031 402
- HU MENGJIE ET AL: "Falsochrobactrum tianjinense sp. nov., a New Petroleum-Degrading Bacteria Isolated from Oily Soils", INTERNATIONAL JOURNAL OF ENVIRONMENTAL RESEARCH AND PUBLIC HEALTH, vol. 19, no. 18, 1 September 2022 (2022-09-01), page 11833, XP093083519, ISSN: 1661-7827, DOI: 10.3390/ijerph191811833
- Chai Lu-jun; Jiang Xia-wei; Zhang Fan; Zheng Bei-wen; Shu Fu-chang; Wang Zheng-liang; Cui Qing-feng; Dong Han-ping; Zhang Zhong-zh: "Isolation and characterization of a crude oil degrading bacteria from formation water: comparative genomic analysis of environmentalOchrobactrum intermediumisolate versus clinical strains", Science B: International Biomedicine & Biotechnology, Zheijiang University Press, CN, vol. 16, no. 10, 14 October 2015 (2015-10-14), pages 865-874, XP036194991, CN ISSN: 1673-1581, DOI: 10.1631/jzus.B1500029
- DATABASE NUCLEOTIDE 28 August 2018 (2018-08-28), "Falsochrobactrum sp. strain HN4 16S ribosomal RNA gene, partial sequence", XP055806574, retrieved from NCBI Database accession no. MH789467

## Description

### TECHNICAL FIELD

The present application relates to the technical field of microbial remediation, in particular to a high-efficiency petroleum degrading bacterium TDYN1^{T} and use thereof.

### BACKGROUND OF THE PRESENT INVENTION

Petroleum is one of the most important energy sources for humans, the consumption of which increases with the development of society and the improvement of human living standards, and the exploitation scale of which expands continuously. However, oil leakage accidents occur frequently during the exploitation, transportation, and application of petroleum, causing serious harm to the ecological environment and human health. In particular, ground crude oil produced during the petroleum exploitation process has become the main source of soil contamination. According to statistics, since 2005, the world total petroleum production is about 3×10¹⁰ tons per year, and about 8 million tons of petroleum contaminants enter the environment, most of which enter the soil.

The hazards of petroleum hydrocarbons entering the soil mainly lie in three aspects: 1) a large amount of petroleum sludge is produced, which has stable physical and chemical properties, is difficult to be removed, and has a long residence time, thereby changing the physical and chemical properties of the soil and the structure of organic matters, and destroying the soil structure and the living environment of soil microorganisms and plants; 2) some high-mobility petroleum hydrocarbons (such as benzene, toluene, xylene, etc.) enter the soil and then reach the underground aquifer along with soil moisture, thereby contaminating groundwater; and 3) some high-volatility petroleum hydrocarbons may be volatilized and diffused into the air after entering the soil, thereby affecting the air quality, and then affecting the human health via the soil, water, and atmospheric environment.

The current methods for treating petroleum-contaminated soil mainly include physical, chemical, and biological remediation technologies. The physical remediation technology mainly refers to physical migration of petroleum hydrocarbon contaminants for the purpose of soil remediation, and is widely used in heavily contaminated soil such as sludge; Wang Zhenguo et. al employs a gas phase extraction method to achieve the petroleum hydrocarbon removal rate reach 88%, in which case the remediation effect is good and no secondary contamination occurs, but the method requires a relatively high remediation cost and is mainly applicable to heavily contaminated soil. The chemical remediation technology refers to injection of chemical oxidants into the soil, using the oxidative properties of the chemical oxidants to remediate petroleum-contaminated soil. Lu et al. uses a H₂O₂-Fe³⁺-EDTA complex to treat petroleum-contaminated soil, to reduce the oil mass ratio in the soil from 14800 mg/kg to 2300 mg/kg, in which case the remediation effect is good and the cost is low, but the chemical agents are easy to cause secondary contamination to the soil.

In recent years, with the development of microbial technologies, the technology of finding high-efficiency degradation strains from in-situ soil to remediate petroleum hydrocarbon contamination is gradually developed. Because of the advantages such as high remediation efficiency, low cost, and environmental friendliness, this technology becomes a development trend of the future soil remediation industry. There are more than 70 genera and 200 species of microorganisms that are known to degrade petroleum, including 28 genera of bacteria such as Pseudomonas genus, Acinetobacter genus, and Achromobacter genus, 30 mold genera such as Penicillium genus, Aspergillus genus, and Fusarium genus, and 12 yeast genera such as Candida genus and Rhodotorula genus. For example, document CN 104805044 A discloses a petroleum-degrading bacterium AH07 strain (Ochrobactrum sp.) isolated from a petroleum contaminated area.

Currently, the degradation rates of existing petroleum degrading bacteria are mostly between 60% and 80%, and the low remediation efficiency becomes a technical bottleneck in the further development and application of this technology. Therefore, the discovery of novel and high-efficient petroleum hydrocarbon degrading bacteria has great prospective significance in improving microbial soil remediation.

### SUMMARY OF THE PRESENT INVENTION

The object of the present application is to provide a high-efficiency petroleum degrading bacterium TDYN1^{T}, to overcome the defect of an insufficient degradation rate of petroleum degrading bacteria in the prior art.

The second object of the present application is to provide use of the high-efficiency petroleum degrading bacterium TDYN1^{T}.

The technical solutions of the present application are summarized as follows:

A high-efficiency petroleum degrading bacterium TDYN1^{T}, identified as Falsochrobactrum sp., is deposited in the China General Microbiological Culture Collection Center, with a deposit number CGMCC No. 18061.

Use of the high-efficiency petroleum degrading bacterium TDYN1^{T} in degrading petroleum in soil.

Preferably, the use of above is operated as follows: immobilizing a bacteria liquid in a logarithmic growth phase of the high-efficiency petroleum degrading bacterium TDYN1^{T} with the deposit number CGMCC No.18061 by means of grass carbon powder, to obtain a remediation bacteria agent with a bacteria density of 10⁸/g, mixing the remediation bacteria agent with 10 kg of petroleum-contaminated soil, the added remediation bacteria agent is 3% by mass, for a reaction at the temperature of 30±1 °C and at the pH ranging from 7 to 7.4.

The present application has the following advantages:
The high-efficiency petroleum degrading bacterium TDYN1^{T} of the present application is used in petroleum-contaminated soil for degrading, having a better effect, in particular, having a high degradation efficiency when the petroleum is degraded at the temperature of 30±1 °C and at the pH value ranging from 7 to 7.4.

### BRIEF DESCRIPTION OF THE DRAWINGS

FIG. 1 is a diagram of the morphology of a strain Falsochrobactrum sp. TDYN1^{T}.
FIG. 2 is a scanning electron microscope photograph of the strain Falsochrobactrum sp. TDYN1^{T}.
FIG. 3 is a degradation curve of strain Falsochrobactrum sp. TDYN1^{T} with respect to temperature.
FIG. 4 is a degradation curve of strain Falsochrobactrum sp. TDYN1^{T} with respect to day.
FIG. 5 is a degradation curve of strain Falsochrobactrum sp. TDYN1^{T} with respect to pH.
FIG. 6 is a petroleum standard curve in a petroleum degradation performance test experiment.

### DETAILED DESCRIPTION OF THE PRESENT INVENTION

Given the petroleum-contaminated soil contamination situation in Tianjin, a strain of high-efficiency petroleum degrading bacteria is found from soil in Tianjin Dagang Oilfield, and a further study for the degradation ability thereof is performed, with a prospect of use thereof to petroleum-contaminated soil.

The method of the present application is described below via specific embodiments. Unless otherwise specified, the technical means used in the present application are all methods known to those skilled in the art. In addition, the embodiments should be construed to be illustrative rather than limiting the scope of the present application, and the essence and scope of the present application are only defined by the claims. For those skilled in the art, without departing from the essence and scope of the present application, various changes or modifications to these embodiments shall also fall into the protection scope of the present application.

### Embodiment 1

### 1. Preparation of materials

A sample of petroleum-contaminated soil was taken from a position in the Tianjin Dagang Oilfield, stored at the temperature of 4°C, and transported back to the laboratory.

### 2. Experimental medium

Inorganic salt medium: distilled water was added to 1 g KH₂PO₄, 0.5 g K₂HPO₄, 10 g NaCl, 1.5g (NH₄)₂SO₄, 0.1 g anhydrous CaCl₂, 0.01 g FeSO₄·7H₂O, and 0.2 g MgSO₄ until the total volume reaches 1000 mL and pH 7.0-7.4, and sterilization was performed at the temperature of 121°C for 20 min.

LB (Luria-Bertani) liquid medium: distilled water was added to 3 g beef extract, 10 g peptone, and 5 g NaCl until the total volume reaches 1000 mL and pH reaches 7.0-7.4, and sterilization was performed at the temperature of 121°C for 20 min.

Slant storage medium and LB solid plate medium: distilled water was added to 5 g beef extract, 10 g peptone, and 5 g NaCl, 20 g agar until the total volume reaches 1000 mL and pH reaches 7.0-7.4, sterilization was performed at 121°C for 20 min, and taken-out was performed at the temperature of 70-80°C.

### 3. Experimental instruments and equipment

Constant temperature oscillator
Electro-heating incubator
Autoclave
Ultraviolet-visible spectrophotometer
Clean bench

### 4. Isolation, screening, and domestication of strains

(1) 10 g of the petroleum-contaminated soil in step 1 was put into a conical flask containing 100 mL LB liquid medium, the conical flask was shaken on a constant temperature shaker at the temperature of 30±1 °C and under the speed of 180 rpm, and enrichment culture was performed for 24 h;
(2) After 24 h, 2 mL culture solution was spread on the LB solid plate medium using a spreading rod, and the LB solid plate medium was put into the incubator at the temperature of 30±1°C, and invert culture was performed for 7 days.
(3) After colonies appear, observation for the morphology of the colonies was performed, colonies with large morphological differences were selected from the LB solid plate medium using a sterile inoculation loop and inoculated on the LB solid plate medium by means of the plate streaking method, invert culture was performed in the incubator at the temperature of 30±1°C for 24 h, after colonies appear, a single colony was selected and inoculated to the LB solid plate medium in the same way; the above streaking process was repeated until a purified colony with a single morphology is formed; and a single colony was stored on the slant storage medium at the temperature of 4°C for later use, which needs to be activated in the LB liquid medium before use.
(4) A petroleum degradation test was performed on the purified colonies separately, 3 ml bacterial liquid was inoculated in 100 ml inorganic salt medium containing 0.1 g crude oil, degradation was performed for 7 days under the conditions of 30°C, 180 rpm, and pH value of 7.2, the remaining crude oil was extracted with petroleum ether, a supernatant was diluted, the absorbance of which was measured by means of ultraviolet spectrophotometry, and a strain degradation rate was determined in comparison with a standard curve; and during the experiment process, a high-efficiency petroleum degrading bacterium Falsochrobactrum sp. TDYN1^{T} was selected, and degradation conditions were measured. Refer to step 5 for details.

### 5. Petroleum degradation performance test experiment for high-efficiency petroleum degrading bacterium Falsochrobactrum sp. TDYN1^{T}

(1) Materials: crude oil, petroleum ether (extractant), LB liquid medium, inorganic salt medium
(2) Method: Ultraviolet spectrophotometry
(3) Steps:

### 1) Preparation of a petroleum standard curve

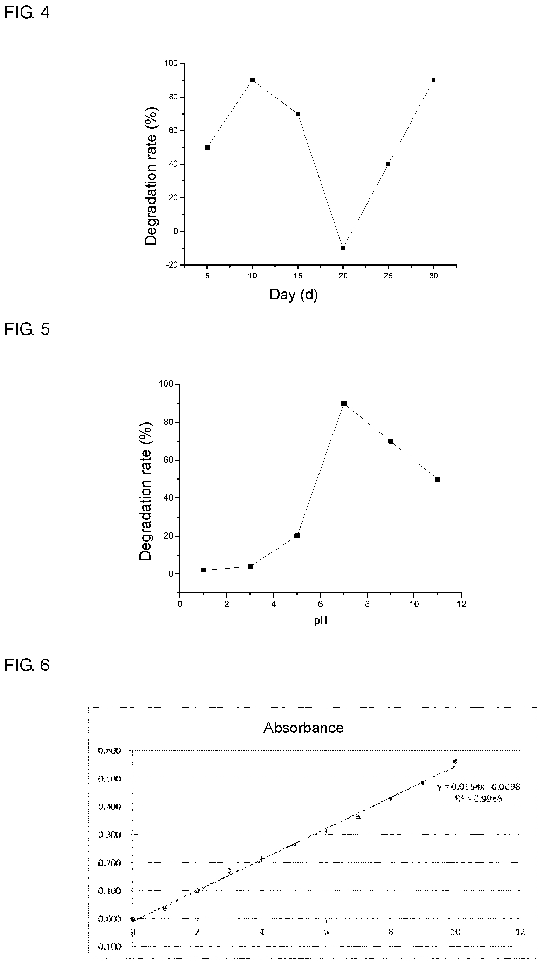

0.1 g petroleum was added to a petroleum ether solution to obtain a constant volume in a 100 mL volumetric flask, after multiple times of dilution, a 25 mg/L petroleum-petroleum ether solution was prepared, and a maximum absorption wavelength is determined to be 256 nm at said concentration; in addition, petroleum-petroleum ether standard solutions with concentrations of 10 mg/L, 20 mg/L, 30 mg/L, 40 mg/L, 50 mg/L, 60 mg/L, 70 mg/L, 80 mg/L, 90 mg/L, and 100 mg/L are prepared, the absorbance is measured at the maximum absorption wavelength, and a standard curve is drawn (as shown in FIG. 6).

### 2) Petroleum degradation

### i. Degradation effects of the strain at different temperatures

3 mL activated bacterial solution was inoculated into 100 mL inorganic salt medium containing 0.1 g petroleum, the process was performed in a sterile environment, and the inoculated solution was separately shaken at constant temperatures 10°C, 15°C, 20°C, 25°C, 30°C, 35°C, 40°C, 45°C, and 50°C under the condition of 180 rpm for 7 d. In addition, three sets of reproducibility tests were performed.

| | | | | | | | | | |
|---|---|---|---|---|---|---|---|---|---|
| Temperature (°C) | 10 | 15 | 20 | 25 | 30 | 35 | 40 | 45 | 50 |
| Degradation rate (%) | 2 | 10 | 40 | 60 | 90 | 85 | 80 | 50 | 10 |

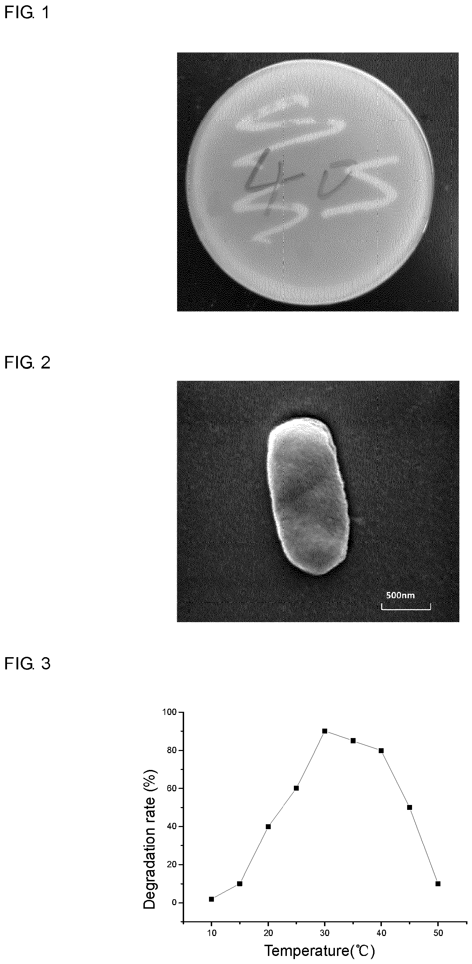

Refer to FIG. 3 for details.

### ii. Degradation effects of the strain at different times

3 mL activated bacterial solution was inoculated into 100 mL inorganic salt medium containing 0.1 g petroleum, the process was performed in a sterile environment, and the inoculated solution was separately shaken at a constant temperature 30°C under the condition of 180 rpm for 5 d, 10 d, 15 d, 20 d, 25 d, and 30 d. In addition, three sets of reproducibility tests were performed.

| | | | | | | |
|---|---|---|---|---|---|---|
| Day (d) | 5 | 10 | 15 | 20 | 25 | 30 |
| Degradation rate (%) | 50 | 90 | 70 | -10 | 40 | 90 |

Refer to FIG. 4 for details.

iii. 3 mL activated bacterial solution was inoculated into 100 mL inorganic salt medium containing 0.1 g petroleum, the pH value of the inorganic salt medium was separately regulated to 1, 3, 5, 7, 9, and 11 before sterilization, the process was performed in a sterile environment, and the inoculated solution was shaken at a constant temperature 30°C at 180 rpm for 7 days. In addition, three sets of reproducibility tests were performed.

| | | | | | | |
|---|---|---|---|---|---|---|
| pH | 1 | 3 | 5 | 7 | 9 | 11 |
| Degradation rate (%) | 2 | 4 | 20 | 90 | 70 | 50 |

Refer to FIG. 5 for details.

### Measurement of the petroleum degradation rate

The degraded solution was extracted twice with 10 mL petroleum ether, a total supernatant was used to measure the absorbance, and the degradation rate was determined in comparison the standard curve.

6. FIG. 1 shows the strain morphology.

7. FIG. 2 shows scanning electron microscope observation of the strain.

### 8. Identification of the strain

The 16S rDNA gene sequence analysis method was used to identify the genus and species of the isolated and screened Falsochrobactrum sp. strain TDYN1^{T}. A 16S rDNA gene sequence (SEQ ID NO.1) has homology reaching 98% with the sequence of the Falsochrobactrum sp. strain HN4 in the NCBI database. An entrustment was given to the China General Microbiological Culture Collection Center to for biochemical identification, which indicates that biochemical characteristics thereof are similar to those of the Falsochrobactrum sp. strain HN4. In combination with the molecular biology and a biochemical identification result, the Falsochrobactrum sp. TDYN1^{T} was identified to be Falsochrobactrum sp.

Full gene sequence of the Falsochrobactrum sp. TDYN1^{T}: (SEQ ID NO.1)

TGCAGTCGACGAGCCCTTCGGGGTTAGTGGCAGACGGGTGAGTAAT
GCATGGGAATCTACCGTTCTCTACGGAATAACTCAGGGAAACTTGTGCTAATA
CCGTATACGCCCTTTTGGGGAAAGATTTATCGGGGAACGATGAGCCCATGTTG
GATTAGCTAGTTGGTGGGGTAAAGGCCCACCAAGGCGACGATCCATAGCTGG
TCTGAGAGGATGATCAGCCACACTGGGACTGAGACACGGCCCAGACTCCTA
CGGGAGGCAGCAGTGGGGAATATTGGACAATGGGCGCAAGCCTGATCCAGC
CATGCCGCGTGAGTGATGAAGGCCTTAGGGTTGTAAAGCTCTTTCACCGGTG
AAGATAATGACGGTAACCGGAGAAGAAGCCCCGGCTAACTTCGTGCCAGCAG

CCGCGGTAATACGAAGGGGGCTAGCGTTGTTCGGAATTACTGGGCGTAAAGC
GCGCGTAGGCGGATTGGTCAGTTAGGGGTGAAATCCCAGGGCTCAACCCTG
GAACTGCCTTTAATACTGCCAGTCTTGAGTATGGAAGAGGTGAGTGGAATTCC
GAGTGTAGAGGTGAAATTCGTAGATATTCGGAGGAACACCAGTGGCGAATGC
GGCTCACTGGTCCATTACTGACGCTGAGGTGCGAAAGCGTGATGAGCAAACA
GGATTAGATACCCTGGTAGTCCACGCCGTAAACTATGAATGTTAGCCGTCGGG
GGGTTTACCTTTCGGTGGCGCAGCTAACGCATTAAACATTCCGCCTGGGGAG
TACGGTCGCAAGATTAAAACTCAAAGGAATTGACGGGGGCCCGCACAAGCG
GTGGAGCATGTGGTTTAATTCGAAGCAACGCGCAGAACCTTACCAGCCCTTG
ACATACCGGTCGCGGATCGAGAGATCGATCTTTCAGTTCGGCTGGACCGGAT
ACAGGTGCTGCATGGCTGTCGTCAGCTCGTGTCGTGAGATGTTGGGTTAAGT
CCCGCAACGAGCGCAACCCTCGCCCTTAGTTGCCAGCATTCAGTTGGGCAC
TCTAAGGGGACTGCCGGTGATAAGCCGAGAGGAAGGTGGGGATGACGTCAA
GTCCTCATGGCCCTTACGGGCTGGGCTACACACGTGCTACAATGGTGGTGAC
AGTGGGCAGCGAGCACGCGAGTGTGAGCTAATCTCCAAAAACCATCTCAGTT
CGGATTGCACTCTGCAACTCGAGTGCATGAAGTTGGAATCGCTAGTAATCGC
GGATCAGCATGCCGCGGTGAATACGTTCCCGGGCCTTGTACACACCGCCCG
TCACACCATGGGAGTTGGTTTTACCTAGAAGGCGCTGTGCTAACCGCAAGGA
GGCAGGCGACCACG

9. Conclusion: in the present application, a petroleum degrading bacterium was isolated and domesticated using a conventional method, and the high-efficiency petroleum degrading bacterium TDYN1^{T} found from the soil in the Tianjin Dagang Oilfield is short rod-shaped, the degradation rate of which reaches 90% after 7 days of petroleum degradation.

### 10. Preservation of the strain:

The high-efficiency petroleum degrading bacterium TDYN1^{T} strain was classified and named as Falsochrobactrum sp., which is a non-spore forming, gram-negative, and short rod bacterium.

Deposit date: July 2, 2019.

Depository organization: China General Microbiological Culture Collection Center.

Deposit address: No. 3, Yard No. 1, Beichen West Road, Chaoyang District, Beijing, Institute of Microbiology, Chinese Academy of Sciences.

Deposit number: CGMCC No.18061. Survival.

### Embodiment 2

### Characteristics of the high-efficiency petroleum degrading bacterium TDYN1^{T}

1. Colony characteristics: seen from FIGs. 1, 2, after inoculated on the LB solid plate medium and cultured at the temperature of 30°C for 24 hours, the colony is milky white, smooth, flat, has neat edges, is gram-negative, and the bacterium is short rod-shaped.
2. Oxidase activity: a piece of filter paper was placed on a clean petri dish, a 1% dimethyl p-phenylenediamine hydrochloride aqueous solution and a 1% a-phenolphthalein alcohol (95%) solution in the same amount were mixed uniformly and then dripped onto the filter paper, the dripping amount is appropriate to make the filter paper wet, and a small amount of a lawn cultured for 18-24 h was picked up using a platinum wire inoculation loop (nickel-chromium wire is inapplicable) and smeared onto the filter paper (the smeared lawn presenting rose red or blue within 10 s is considered to be positive, presenting color in 10-60 s is a delayed reaction, and the smeared lawn presenting color after 60 s is considered to be negative).

Test observation: a color reaction occurs in 15 s, indicating a positive reaction.

### 3. Catalase activity:

1) Reagent preparation: 3%-10% hydrogen peroxide.
2) Inoculation and result observation: a platinum wire inoculation loop was adopted to smear a small loop of slant bacteria cultured for 24 h onto a glass slide that was dripped with 3% hydrogen peroxide, observation as to whether there are bubbles generated is performed after standing for 1-3 minutes, if there are bubbles generated, the result is positive, and if there is no bubble generated, the result is negative. Test observation: there are bubbles generated, indicating a positive result.

4. Sugar fermentation test: according to the difference in the ability of the bacterium to decompose and utilize sugar, the production of acid and gas is used as the basis for identification. A specific operation is as follows: an indicator bromomethyl violet (B.C.P indicator, presenting yellow when the pH value is below 5.2, and presenting purple when the pH value is above 6.8) was added to a sugar fermentation medium, and determination was performed according to a color change of the indicator after cultivation. Observation as to whether there is gas generated can be performed by putting an inverted Dulbecco tube into the fermentation medium.
1) Preparation of the sugar fermentation medium: 2 g peptone and 5 g NaCl were dissolved in hot water, the pH value was regulated to 7.4, 0.2 g of K₂HPO₄ was added, distilled water was added until the total volume reaches 1000 ml, and then 3 ml of 1% bromothymol blue aqueous solution (the 1% aqueous solution was prepared by first dissolving bromothymol blue with a small amount of 95% ethanol and then adding water thereto) was added. Glucose and lactose were respectively added to two parts of the above prepared solution; the two parts of the solution were filled into test tubes separately, with a filling volume of 4-5 cm height, then poured into a Duchenne tubule (the mouth of the tubule faces downward, and the tubule is filled with a culture solution), and sterilized by moist heat at the temperature of 115°C for 20 min. During sterilization, a boiling time should be prolonged appropriately, to exhaust cold air as much as possible such that no bubble remains in the Duchenne tubule.
2) Test tube labeling: a blank control was labeled for each type of sugar fermentation.
3) Inoculation culture: a small amount of a lawn was respectively inoculated to a corresponding test tube using an aseptic technique, and the blank control for each type of sugar fermentation is not inoculated with bacteria. The Dulbecco tube was inverted and placed into a test tube, for cultivation at a constant temperature of 30°C, and observation was performed at 24 h, 36 h, and 72 h respectively to obtain results.
4) Observation record: compared with the blank control tube, if the culture solution in the inoculation tube maintains an original color, a reaction result is negative, indicating that the bacterium cannot utilize this type of sugar, and a record is represented by "-"; if the culture solution becomes yellow, a reaction result is positive, indicating that the bacterium can decompose this type of sugar to produce acid, and a record is represented by "+". If bubbles are generated in the Duchenne tubule in the culture solution, it is a positive reaction, indicating that the bacterium can decompose this type of sugar to produce acid and gas, and the record is represented by "+". If no bubble is generated in the Duchenne tubule, it is a negative reaction, and the record is represented by "-".

Test observation: the bacterium can utilize glucose and lactose, in both cases producing acid without producing a gas.

### 5. Ammonia production test:

1) Preparation of ammonia producing medium: distilled water was added to 5 g peptone until the total volume reaches 1000 ml and pH value reaches 7.2, the solution was divided and put into test tubes separately, and sterilized at 121°C for 15-20 min.
2) Reagent preparation: 20 g potassium iodide was dissolved in 50 ml water, small mercury iodide particles were added to this solution until the solution is saturated (about 32 g), then 460ml water and 134 g potassium hydroxide were added, and a supernatant was stored in a dark bottle for later use.
3) Inoculation: a fresh strain was inoculated and cultivated at an appropriate temperature for 1, 3, and 5 days.
4) Result observation: a few drops of the reagent was added to a small amount of the culture solution, and the production of a yellowish-brown precipitate indicates a positive result.

Test observation: a yellowish-brown precipitate is produced, indicating a positive reaction.

### 6. Indole test:

1) Preparation of an indole medium: distilled water was added to10 g peptone and 5g NaCl until the total volume reaches 1000 ml and pH reaches 7.2-7.4, and sterilization was performed with moist heat at 121°C for 20 min.
2) Preparation of an indole reagent: 8 g p-xylaminobenzaldehyde, 760 ml ethanol (95%), and 160 ml concentrated HCl.
3) Test tube labeling: the indole medium was put into a corresponding test tube, and a blank control was labeled.
4) Inoculation and culture: a small amount of a lawn was respectively inoculated into a corresponding test tube using an aseptic technique, a test tube was used as a blank control without inoculation, and cultivation was performed in an incubator at a constant temperature of 30° C for 24-48 h.
5) Observation records: a culture solution was taken out, 3-5 ml indole reagent was added slowly to the surface of the culture solution along the tube wall, and if a red color appears at the interface of the liquid layer, it is a positive reaction. If the color is not obvious, 4-5 drops of ether can be added to the culture solution, the culture solution was shaken to disperse the ether in the liquid, then the culture solution stood for a while, and the indole reagent was added after the ether floats to the surface. If there is indole in the culture solution, the indole can be extracted into an ether layer, the concentrated indole reacts with the reagent, and the color is obvious. If there is indole, the ether presents a rose red color, indicating a positive reaction to the indole test, and a record is represented by "+"; and if there is no indole, the ether presents an original color, indicating a negative reaction to the indole test, and a record is represented by "-".

Test observation: a red color appears at the interface, indicating a positive reaction.

The characteristics of the high-efficiency degrading bacterium TDYN1^{T} are summarized in the following table:

| Test type | | | | Property |
|---|---|---|---|---|
| | oxidase | | | "+" |
| | catalase | | | "+" |
| | | glucose | acid production | "+" |
| sugar fermentation | | | gas production | "-" |
| | | lactose | acid production | "+" |
| | | | gas production | "-" |
| ammonia production | | | | "+" |
| | indole | | | "+" |

### Embodiment 3

Use of the high-efficiency petroleum degrading bacterium TDYN1^{T} to degrading of petroleum in soil

A bacteria liquid in a logarithmic growth phase of the high-efficiency petroleum degrading bacterium TDYN1^{T} with the deposit number CGMCC No.18061 is immobilized by means of grass carbon powder, to obtain a remediation bacteria agent with a bacteria density of 10⁸/g, and the remediation bacteria agent is mixed with 10 kg petroleum-contaminated soil ( from the Tianjin Dagang Oilfield), the added remediation bacteria agent is 3% by mass, for a reaction under the conditions of 30°C and pH value of 7.2 for 10 days, with a petroleum degradation rate of 81%.

### Embodiment 4

Use of the high-efficiency petroleum degrading bacterium TDYN1^{T} to degrading of petroleum in soil

A bacteria liquid in a logarithmic growth phase of the high-efficiency petroleum degrading bacterium TDYN1T with the deposit number CGMCC No.18061 is immobilized by means of grass carbon powder, to obtain a remediation bacteria agent with a bacteria density of 10⁸/g, and the remediation bacteria agent is mixed with 10 kg petroleum-contaminated soil ( from the Tianjin Dagang Oilfield), the added remediation bacteria agent is 3% by mass, for a reaction under the conditions of 29°C and pH 7 for 15 days, with a petroleum degradation rate of 85%.

### Embodiment 5

Use of the high-efficiency petroleum degrading bacterium TDYN1^{T} to degrading of petroleum in soil

A bacteria liquid in a logarithmic growth phase of the high-efficiency petroleum degrading bacterium TDYN1^{T} with the preservation number CGMCC No.18061 is immobilized by means of grass carbon powder, to obtain a remediation bacteria agent with a bacteria density of 10⁸/g, and the remediation bacteria agent is mixed with 10 kg petroleum-contaminated soil ( selected from the Tianjin Dagang Oilfield), the added remediation bacteria agent is 3% by mass, for a reaction under the conditions of 31°C and pH 7.4 for 12 days, with a petroleum degradation rate of 83%.

## Claims

1. A high-efficiency petroleum degrading bacterium TDYN1^{T}, identified as Falsochrobactrum sp., is deposited in the China General Microbiological Culture Collection Center, with a deposit number CGMCC No. 18061.

2. Use of the high-efficiency petroleum degrading bacterium TDYN1^{T} according to claim 1 in degrading petroleum in soil.

3. The use of the high-efficiency petroleum degrading bacterium TDYN1^{T} according to claim 2, **characterized in that**, the use comprising the following steps: immobilizing a bacteria liquid in a logarithmic growth phase of the high-efficiency petroleum degrading bacterium TDYN1^{T} with the deposit number CGMCC No.18061 by means of grass carbon powder, to obtain a remediation bacteria agent with a bacteria density of 10⁸/g, mixing the remediation bacteria agent with 10 kg of petroleum-contaminated soil, the added remediation bacteria agent is 3% by mass, for a reaction at the temperature of 30±1 °C and at the pH ranging from 7 to 7.4.

4. The high-efficiency petroleum degrading bacterium TDYN1 ^{T} according to claim 1, **characterized in that** a 16S DNA sequence of the petroleum degrading bacterium TDYN1^{T} is as shown in SEQ ID NO.1.

## Patentansprüche

1. Hocheffizientes erdölabbauendes Bakterium TDYN1^{T}, identifiziert als Falsochrobactrum sp., ist in dem China General Microbiological Culture Collection Center mit einer Hinterlegungsnummer CGMCC Nr. 18061 hinterlegt.

2. Verwendung des hocheffizienten erdölabbauenden Bakteriums TDYN1^{T} nach Anspruch 1 zum Abbauen von Erdöl im Boden.

3. Verwendung des hocheffizienten erdölabbauenden Bakteriums TDYN1^{T} nach Anspruch 2, **dadurch gekennzeichnet, dass** die Verwendung die folgenden Schritte umfasst: Immobilisieren einer Bakterienflüssigkeit in einer logarithmischen Wachstumsphase des hocheffizienten erdölabbauenden Bakteriums TDYN1^{T} mit der Hinterlegungsnummer CGMCC Nr. 18061 mittels Graskohlenstoffpulver, um ein bakterielles Sanierungsmittel mit einer Bakteriendichte von 10⁸/g zu erlangen, Mischen des bakteriellen Sanierungsmittels mit 10 kg erdölkontaminiertem Boden, wobei das hinzugefügte bakterielle Sanierungsmittel 3 Massenprozent ist, für eine Reaktion bei einer Temperatur von 30 ±1 °C und bei dem pH in einem Bereich von 7 bis 7,4.

4. Hocheffizientes erdölabbauendes Bakterium TDYN1^{T} nach Anspruch 1, **dadurch gekennzeichnet, dass** eine 16S-DNA-Sequenz des Erdöl abbauenden Bakteriums TDYN1^{T} wie gezeigt in SEO ID NO. 1 ist.

## Revendications

1. Bactérie haute performance dégradant le pétrole TDYN1^{T}, identifiée en tant que Falsochrobactrum sp., déposée au Centre Général de Collecte de Cultures Microbiologiques de Chine avec le numéro de dépôt CGMCC N° 18061.

2. Utilisation de la bactérie haute performance dégradant le pétrole TDYN1^{T} de la revendication 1 dans la dégradation du pétrole dans le sol.

3. Utilisation de la bactérie haute performance dégradant le pétrole TDYN1^{T} selon la revendication 2, **caractérisée en ce que** l'utilisation comprend les étapes suivantes : immobilisation d'un liquide bactérien en phase de croissance logarithmique de la bactérie haute performance dégradant le pétrole TDYN1^{T} ayant le numéro de dépôt CGMCC N° 18061 au moyen d'une poudre de carbone de graminées, pour que soit obtenu un agent bactérien d'assainissement ayant une densité de bactéries de 10⁸/g, mélange de l'agent bactérien d'assainissement avec 10 kg de sol contaminé par du pétrole, l'agent bactérien d'assainissement étant présent à raison de 3 % en masse, pour une réaction à une température de 30 ± 1°C et à un pH situé dans la plage allant de 7 à 7,4.

4. Bactérie haute performance dégradant le pétrole TDYN1^{T} selon la revendication 1, **caractérisée en ce que** la séquence d'ADN 16S de la bactérie dégradant le pétrole TDYN1^{T} est telle que présentée dans la SEQ ID NO : 1.
